# EUROPEAN PATENT APPLICATION

(11) **EP 1 318 150 A1**
(43) Date of publication of application: **11.06.2003**
(21) Application number: 01963508.5
(22) Date of filing: 07.09.2001
(51) Int. Cl.: C07D 413/12

(54) **NOVEL CRYSTALS OF 1,3,4-OXADIAZOLE DERIVATIVE, PROCESS FOR PRODUCING THE CRYSTALS AND MEDICINES CONTAINING THE SAME AS THE ACTIVE INGREDIENT**

(30) Priority: 08.09.2000 JP 2000272437
(71) Applicant: Ono Pharmaceutical Co., Osaka 541-8526 (JP)
(72) Inventor: KOJIMA, Tsutomu Fukui Res. Inst. ONO Ph. Co,ltd, Sakai-gun,Fukui 913-003 (JP); OHMOTO, Kazuyuki Min. Res. Inst. ONO Ph. Co., Ltd, Osaka 618-8585 (JP)
(74) Representative: Henkel, Feiler, Hänzel
(86) International application number: JP0107774
(87) International publication number: WO02020516

(57) **Abstract**

Type-2 crystals of 2-[5-amino-6-oxo-2-phenyl-1,6-dihydro-1-pyrimidyl]-N-[1-(2-[5-t-butyl-1,3,4-oxadiazolyl]carbonyl)-2-(R, S)-methylpropyl]acetamide represented by formula (I), a process for producing the same, and a pharmaceutical agent containing the same as an active ingredient.

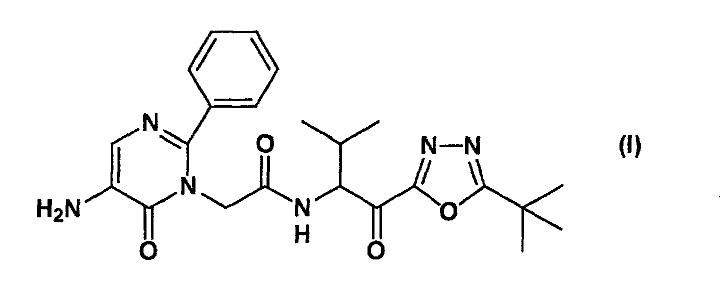

## Description

### Technical Field

The present invention relates to novel crystals of 2-[5-amino-6-oxo-2-phenyl-1,6-dihydro-1-pyrimidyl]-N-[1-(2-[5-t-butyl-1,3,4-oxadiazolyl]carbonyl)-2-(R,S)-methylpropyl]acetamide which is useful as a medicament. More specifically, the present invention relates to novel Type-2 crystals of 2-[5-amino-6-oxo-2-phenyl-1,6-dihydro-1-pyrimidyl]-N-[1-(2-[5-t-butyl-1,3,4-oxadiazolyl]carbonyl)-2-(R, S)-methylpropyl]acetamide represented by formula (I): 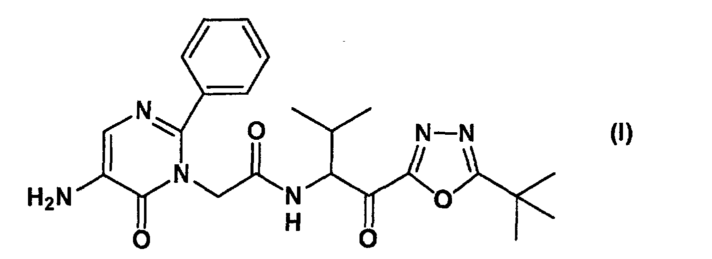 a process for producing the same, and a pharmaceutical agent comprising the same as an active ingredient.

### Background Art

In Example 113 of WO98/24806 it is disclosed that the compound represented by formula (I) has an inhibitory activity against a serine protease (especially, elastase) and is useful for treating and/or preventing diseases relating to an elastase, e.g., arthritis, periodontal disease, nephritis, dermatitis, psoriasis, cystic fibrosis, chronic bronchitis, atherosclerosis, Alzheimer's disease, organ transplantation, corneal ulcers, and malignant tumors.

However, in the above Description, there is not description about crystals of the compound of the formula (I), and there is not description about crystal polymorphism, too at all.

In general, required conditions for medicaments include effectiveness, safety, and maintenance of the same quality. That is, even when a compound has an excellent efficacy and a confirmed safety, the compound may invite harmful contingencies and has no justification for existence as a medicaments unless a constant quality can be maintained at actual production and distribution. Thus, in a medicaments, it is an extremely important problem to maintain a constant quality. For maintaining a constant quality, it is necessary to provide the drug substance always having the same quality. For that purpose, it is very effective to provide the drug substance in a stable crystalline state.

It is known that a compound exhibiting polymorphism generally affords different crystals forms depending on recrystallization methods and the difference in crystal form results in different solubility, dissolution rate, stability, absorbability, etc., which may sometimes cause different pharmacological effects.

Therefore, for employing a compound exhibiting polymorphism as a medicament, it is necessary to have always a constant and stable crystal form at the production and storage and thus it is crucial to find such a stable crystal form.

When the present inventors have prepared the compound represented by formula (I) in accordance with the method described in Example 113 of WO98/248 they have found that the resulting compound is not a compound having a single crystal form. In addition, it is difficult to separate impurities such as a solvent from the compound thus obtained and it is extremely difficult to obtain the compound always having a constant quality in good reproducibility.

That is, the compound obtained by the method described in Description of WO98/24806 is very difficult to provide in a large quantity as a stable drug substance for a medicament which always has a constant quality, and hence possesses a serious problem for practical use as a medicament.

Accordingly, it is desired to provide the compound represented by formula (I) or a salt thereof having a single stable crystal form, which always has a constant quality and can be provided in a large quantity as the drug substance for a medicament.

### Disclosure of the Invention

Therefore, with regard to 2-[5-amino-6-oxo-2-phenyl-1,6-dihydro-1-pyrimidyil]-N-[1-(2-[5-t-butyl-1,3,4-oxadiazolyl]carbonyl)-2-(R, S)-methylpropyl]acetamide and salts thereof, the present inventors have intensively studied for the purpose of providing a single and stable crystal form capable of providing the drug substance for a medicament always having a constant quality in a large quantity.

First, as a result of studies on various acid addition salts (hydrochloride, sulfate, methanesulfonate, p-toluenesulfonate, maleate, lactate, malate, tartrate, and phosphate), the inventors have found that only the hydrochloride forms crystals. However, it has been found that the hydrochloride precipitates as a free form (Type-2 crystals) upon standing for a while after dissolution in water. For this reason, the hydrochloride is found to be problematic for use in an aqueous solution. Moreover, since the hydrochloride has a possibility of conversion into the free form (Type-2 crystals) or decomposition by the moisture in the air, the salt is also found to be unsuitable for use in a solid preparation.

As a result of extensive studies for obtaining a single crystal form of the free form, the inventors have succeeded in obtaining Type-1 crystals and Type-2 crystals. However, use of tetrahydrofuran as a solvent for recrystallization is needed for providing a large quantity of Type-1 crystals stably in a good reproducibility. When tetrahydrofuran is used, however, it is impossible to remove tetrahydrofuran perfectly even by any drying process and crystals containing tetrahydrofuran are only obtained. Moreover, for removing tetrahydrofuran in this Type-1 crystals and obtaining Type-1 crystals, it is necessary to reflux them in ethanol. In that case, Type-1 crystals containing a large quantity of ethanol instead of tetrahydrofuran is formed. Thus, Type-1 crystals contain a large quantity of a solvent as an impurity in every case. Furthermore, it is found that addition of Type-1 crystals to water promptly results in precipitation of Type-2 crystals. Therefore, Type-1 crystals are found to have not only a problem that it is impossible to remove a solvent perfectly but also a problem that the crystals are extremely problematic for use in an aqueous solution. In addition, since they have a possibility of conversion into the free form (Type-2 crystals) by the moisture in the air, the crystals are also found to be unsuitable for use in a solid preparation.

Thus, Type-1 crystals are unstable and are extremely problematic for use as the drug substance for a medicament.

On the other hand, Type-2 crystals are a single crystal form which can be provided in a large quantity in a good reproducibility, and the solvent used for recrystallization can be removed almost perfectly by drying.

That is, the inventors have found that Type-2 crystals of 2-[5-amino-6-oxo-2-phenyl-1 ,6-dihydro-1-pyrimidyl]-N-[1-(2-[5-t-butyl-1,3,4-oxadiazolyl]carbonyl)-2-(R,S)-methylpropyl]acetamide are single stable crystals, which always have a constant quality and can be provided in a large quantity, and are suitable as the drug substance for a medicament, which could achieve the purpose of the invention.

Type-2 crystals of 2-[5-amino-6-oxo-2-phenyl-1,6-dihydro-1-pyrimidyl]-N-[1-(2-[5-t-butyl-1,3,4-oxadiazolyl]carbonyl)-2-(R, S)-methylpropyl]acetamide according to the present invention have data of X-ray crystal diffraction spectrum, differential scanning calorimetry (DSC), and infrared absorption spectrum, which are clearly different from those of the compound obtained in accordance with the method described in Example 113 of WO98/24806. Therefore, it is found that the crystals are a novel crystals having a different crystal form.

Furthermore, impurities such as a recrystallization solvent can be almost perfectly removed from the novel Type-2 crystals, and the crystals are also excellent in characteristic properties for formulation (solubility, stability, etc.).

That is, the present invention relates to:
(1) a type-2 crystal of 2-[5-amino-6-oxo-2-phenyl-1,6-dihydro-1-pyrimldyl]-N-[1-(2-[5-t-butyl-1,3,4-oxadiazolyl]carbonyl)-2-(R,S)-methylpropyl]acetamide,
(2) a process for producing the same, and
(3) a pharmaceutical agent comprising the same as an active ingredient.

Type-2 crystals of 2-[5-amino-6-oxo-2-phenyl-1,6-dihydro-1-pyrimidyl]-N-[1-(2-[5-t-butyl-1,3,4-oxadiazolyl]carbonyl)-2-(R, S)-methylpropyl]acetamide are characterized by the data of diffraction angle (2θ), half band width, relative intensity shown in the following Table 1 in a powder X-ray diffraction spectrum obtained using Cu-Kα ray.

**Table 1**

| Diffraction angle (2θ) | Half band width | Relative intensity |
|---|---|---|
| 4.60 | 0.09 | Strong |
| 8.52 | 0.14 | Slightly strong |
| 11.64 | 0.17 | Medium |
| 12.16 | 0.19 | Medium |
| 14.28 | 0.19 | Medium |
| 15.52 | 0.14 | Medium |
| 17.48 | 0.19 | Strong |
| 18.72 | 0.12 | Medium |
| 19.82 | 0.21 | Medium |
| 20.62 | 0.14 | Medium |
| 21.30 | 0.24 | Slightly strong |
| 22.54 | 0.17 | Strong |
| 23.08 | 0.12 | Medium |
| 23.56 | 0.07 | Medium |
| 24.08 | 0.07 | Medium |
| 24.20 | 0.12 | Medium |
| 25.10 | 0.17 | Medium |
| 27.30 | 0.12 | Medium |
| 28.62 | 0.14 | Medium |
| 31.00 | 0.07 | Medium |

Type-2 crystals of 2-[5-amino-6-oxo-2-phenyl-1,6-dihydro-1-pyrimidyl]-N-[1-(2-[5-t-butyl-1,3,4-oxadiazolyl]carbonyl)-2-(R,S)-methylpropyl]acetamide of the present invention (hereinafter, sometimes abbreviated as the "compound of the present invention") are specified by the physicochemical properties in the present specification but individual spectral data may vary to some extent from their natures and hence should not be construed strictly.

For example, from the nature of powder X-ray diffraction spectral data, diffraction angle (2θ), half band width, and total pattern are important in the recognition of identity of crystals but relative intensity may vary in some degree depending on the direction of crystal growth, particle size, and measuring conditions.

Moreover, also in differential scanning calorimetric data, total pattern is important in the recognition of identity of crystals but may vary in some degree depending on measuring conditions.

Furthermore, also in infrared absorption spectral data, total pattern is important in the recognition of identity of crystals but may vary in some degree depending on measuring conditions.

### Brief Description of the Drawings

Fig. 1 shows a powder X-ray diffraction spectrum of the compound of the present invention.
Fig. 2 shows differential scanning calorimetric data of the compound of the present invention.
Fig. 3 shows infrared absorption spectral data of the compound of the present invention.
Fig. 4 shows single crystal structural data of the compound of the present invention.
Fig. 5 shows single crystal structural packing data of the compound of the present invention.
Fig. 6 shows an electron microscopic photograph of the compound of the present invention.
Fig. 7 shows powder X-ray diffraction spectral data of the compound of Comparative Example 1
Fig. 8 shows differential scanning calorimetric data of the compound of Comparative Example 1
Fig. 9 shows infrared absorption spectral data of the compound of Comparative Example 1
Fig. 10 shows powder X-ray diffraction spectral data of Type-1 crystals (the compound of Comparative Example 2).
Fig. 11 shows differential scanning calorimetric data of the compound of Comparative Example 2.
Fig. 12 shows infrared absorption spectral data of the compound of the comparative Example 2.
Fig. 13 shows multi-recorded powder X-ray diffraction spectral data of the compound of the present invention, the compound of Comparative Example 1, and the compound of Comparative Example 2.
Fig. 14 shows multi-recorded differential scanning calorimetric data of the compound of the present invention, the compound of Comparative Example 1, and the compound of Comparative Example 2.
Fig. 15 shows multi-recorded infrared absorption spectral data of the compound of the present invention, the compound of Comparative Example 1, and the compound of Comparative Example 2 (1800 to 1200 cm⁻ ¹).
Fig. 16 shows multi-recorded infrared absorption spectral data of the compound of the present invention, the compound of Comparative Example 1, and the compound of Comparative Example 2 (1200 to 600 cm⁻¹).

### Detailed Description of the Invention

Type-2 crystals of 2-[5-amino-6-oxo-2-phenyl-1,6-dihydro-1-pyrimidyl]-N-[1-(2-[5-t-butyl-1,3,4-oxadiazolyl]carbonyl)-2-(R,S)-methylpropyl]acetamide of the present invention can be produced by the following method or the method described in Examples.

That is, the crystals can be produced by recrystallizing a crude purified product of 2-[5-amino-6-oxo-2-phenyl-1,6-dihydro-1-pyrimidyl]-N-[1-(2-[5-t-butyl-1,3,4-oxadiazolyl]carbonyl)-2-(R,S)-methylpropyl]acetamide using a mixed solvent composed of water and an organic solvent miscible with water.

The recrystallization is carried out using, for example, a mixed solvent of water with an organic solvent (one or more solvents selected from the group consisting of ethanol, methanol, dimethylformamide, dimethyl sulfoxide, acetone, and ethyl acetate).

The ratio of the organic solvent to water is preferably from 10:1 to 1:5, more preferably from 3:1 to 1:2.

Moreover, the mixed solvent (organic solvent and water) is preferably used in an amount of 1 mL to 80 mL, more preferably in an amount of 5 mL to 60 mL, per 1 g of the crude purified product.

The crude purified product of 2-[5-amino-6-oxo-2-phenyl-1,6-dihydro-1 -pyrimidyl]-N-[1-(2-[5-t-butyl-1,3,4-oxadiazolyl]carbonyl)-2-(R,S)-methylpropyl]acetamide for use in the production of the compound of the present invention can be produced by subjecting 2-[5-benzyloxycarbonylamino-6-oxo-2-phenyl-1,6-dihydro-1-pyrimidyl]-N-[1-(2-[5-t-butyl-1,3,4-oxadiazolyl]carbonyl)-2-(R, S)-methylpropyl]acetamide represented by formula (II) to a deprotection reaction : 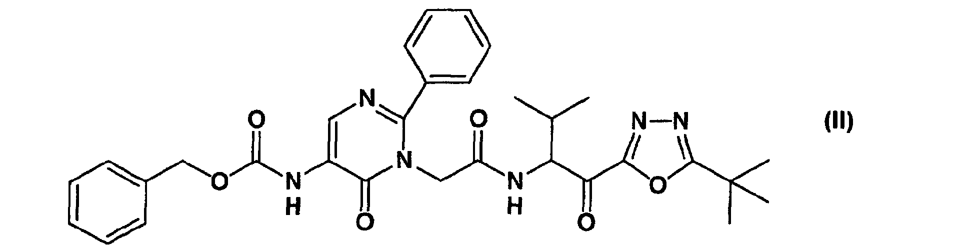 or by subjecting 2-[5-nitro-6-oxo-2-phenyl-1,6-dihydro-1-pyrimidyl]-N-[1-(2-[5-t-butyl-1, 3, 4-oxadiazolyl]carbonyl)-2-(R, S)-methylpropyl]acetamide represented by formula (lll): 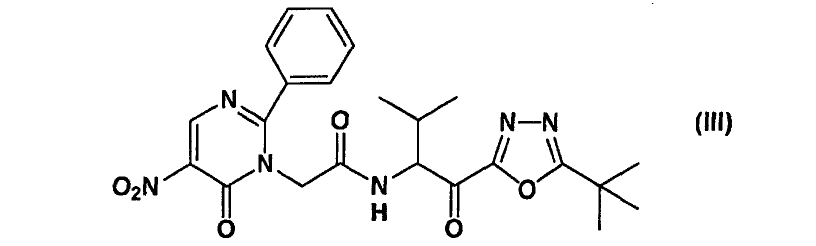 to a reduction reaction.

The deprotection reaction of the compound of formula (II) is known and is carried out by the method described in WO98/2480 and hydrogenolysis reaction.

The hydrogenolysis reaction is known and the deprotection reaction by the hydrogenolysis is carried out, for example, at a temperature of 0 to 200°C under a hydrogen atmosphere or in the presence of ammonium formate under normal pressure or increased pressure, in the presence or absence of an inorganic acid (e.g., hydrochloric acid, sulfuric acid, hypochlorous acid, boric acid, tetrafluoroboric acid, etc.) or an organic acid (e.g., acetic acid, p-toluenesulfonic acid, oxalic acid, trifluoroacetic acid, formic acid, etc.) in the presence of a hydrogenation catalyst (e.g., palladium-carbon, palladium black, palladium, palladium hydroxide, platinum dioxide, nickel, Raney nickel, ruthenium chloride, etc.) in an inert solvent [ether-type (e.g., tetrahydrofuran, dioxane, dimethoxyethane, diethyl ether, etc.), alcohol-type (e.g., methanol, ethanol, etc.), benzene-type (e.g., benzene, toluene, etc.), ketone-type (e.g., acetone, methyl ethyl ketone, etc.), nitrile-type (e.g., acetonitrile etc.), amide-type (e.g., dimethylformamide etc.), water, ethyl acetate, acetic acid, mixtures of two or more of them, or the like]. When an acid is used, a salt thereof may be used.

The reduction reaction of the nitro group of the compound of formula (lll) is known and carried out by, for example, hydrogenolysis reaction or reduction reaction using an organic metal.

The hydrogenolysis reaction is carried out by the method described above.

The reduction reaction using an organic metal is known and is carried out, for example, at a temperature of 50 to 150°C using an organic metal (zinc, iron, tin, tin chloride, iron chloride, *etc.* ) in the presence or absence of an aqueous hydrochloric acid solution in a water-miscible solvent (ethanol, methanol, etc.).

Furthermore, as the crude purified product of 2-[5-amino-6-oxo-2-phenyl-1,6-dihydro-1-pyrimidyl]-N-[1-(2-[5-t-butyl-1, 3,4-oxadiazolyl]carbonyl)-2-(R,S)-methylpropyl]acetamide for use in the production of the compound of the present invention, a product obtained by treatment and concentration after the deprotection or the reduction reaction of a nitro group or a solution before the concentration may be used. However, the solution before the concentration is used only when the solvent for the deprotection or the reduction reaction of a nitro group is a water-miscible organic solvent (one or more solvents selected from the group consisting of ethanol, methanol, dimethylformamide, and dimethyl sulfoxide).

### Best Mode for Carrying Out the Invention

The present invention is described below in detail with reference to Examples but the present invention should not be construed as being limited thereto.

Solvents in parentheses shown in the separation by chromatography mean eluting or developing solvents used and ratios are by volume.

### Example 1 (1): Process for producing Type-2 crystals of 2-[5-amino-6-oxo-2-phenyl-1,6-dihydro-l 1-pyrimidyl]-N-[1-(2-[5-t-butyl-1,3,4-oxadiazolyl]carbonyl)-2-(R,S)-methylpropyl]acetamide

A suspension of 2-[5-amino-6-oxo-2-phenyl-1,6-dihydro-1-pyrimidyl]-N-[1-(2-[5-t-butyl-1,3,4-oxadiazolyl]carbonyl)-2-(R,S)-methylpropyl]acetamide (850 g) in methanol (18 L) was stirred at 50°C for 30 minutes to dissolve the compound. The resulting solution was filtered under heating, followed by washing with methanol (2L). Water (28 L) was added to the solution at an inner temperature of 35°C or lower and the mixture was stirred at about 35°C for about 4 hours, followed by stirring overnight for cooling. The precipitated crystals were filtered and dried *in vacuo* at 60°C for 15 hours to obtain a compound of the present invention (826 g) having the following physical properties.

It was confirmed by gas chromatography that methanol as a residual solvent remained in only 0.24% content in the crystals of the compound of the present invention obtained by the above method. This level of the residual amount was not significant at all in view of medicinal regulation.

### Example 1 (2): Process for producing Type-2 crystal of 2-[5-amino-6-oxo-2-phenyl-1,6-dihydro-1-pyrimldyl]-N-[1-(2-[5-t-butyl-1, 3,4-oxadiazolyl]carbonyl)-2-(R,S)-methylpropyl]acetamide

To a solution of 2-[5-benzyloxycarbonylamino-6-oxo-2-phenyl-1,6-dihydro-1 -pyrimidyl]-N-[1-(2-[5-t-butyl-1,3,4-oxadiazolyl]carbonyl)-2-(R, S)-methylpropyl]acetamide (4.11 kg) in methanol (35 L) was added 10% palladium carbon (50% hydrous, 600 g) at room temperature under an argon atmosphere. The reaction mixture was stirred at room temperature for 20 minutes under a hydrogen atmosphere at 3 atm. The reaction mixture was filtered, followed by washing with methanol (14 L). This operation was repeated once. Two filtrates were combined and concentrated under reduced pressure. After 46 L of methanol was evaporated, methanol (26 L) was added to the concentration residue (crystals precipitated), which was dissolved under heating. The solution was filtered, followed by washing with methanol (6 L). Under stirring, filtered water (39 L) was added to the solution, followed by stirring at room temperature overnight. The mixture was then cooled to an inner temperature of 5°C and stirred for 1 hour. The precipitated crystals were filtered and washed with water (12 L). The resulting crystals were dried at 60°C for 15 hours and further at 80°C for 15 hours under reduced pressure to obtain a compound of the present invention (4.15 kg) having the following physical properties.

It was confirmed by gas chromatography that methanol as a residual solvent remained in only 0.06% content in the crystals of the compound of the present invention obtained by the above method. This level of the residual amount was not significant at all in view of medicinal regulation.

### Example 1 (3): Process for producing Type-2 crystal of 2-[5-amino-6-oxo-2-phenyl-1,6-dihydro-1-pyrimidyl]-N-[1-(2-[5-t-butyl-1,3,4-oxadiazolyl]carbonyl)-2-(R,S)-methylpropyl]acetamide

A mixture of 2-[5-amino-6-oxo-2-phenyl-1,6-dihydro-1-pyrimidyl]-N-[1-(2-[5-t-butyl-1,3,4-oxadiazolyl]carbonyl)-2-(R, S)-methylpropyl]acetamide (900 g)in ethyl acetate (11.6 L), and water (6.3 L) was heated at 65°C to dissolve the compound. The resulting solution was filtered under heating, followed by washing with ethyl acetate (1.1 L). The filtrate was cooled to 18°C over 4 hours, followed by stirring for 40 minutes. The precipitated crystals were filtered, washed with t-butyl methyl ether (900 mL x 3), and dried under reduced pressure at 65°C for 15 hours to obtain a compound of the present invention (635 g) having the following physical properties.

It was confirmed by gas chromatography that ethyl acetate as a residual solvent remained in only 0.39% content in the crystals of the compound of the invention obtained by the above method. This level of the residual amount was not significant at all in view of medicinal regulation.

### Example 1 (4): Process for producing Type-2 crystal of 2-[5-amino-6-oxo-2-phenyl-1,6-dihydro-1-pyrimidyl]-N-[1-(2-[5-t-butyl-1,3,4-oxadiazolyl]carbonyl)-2-(R,S)-methylpropyl]acetamide

A suspension of 2-[5-amino-6-oxo-2-phenyl-1,6-dihydro-1-pyrimidyl]-N-[1-(2-[5-t-butyl-1,3,4-oxadiazolyl]carbonyl)-2-(R,S)-methylpropyl]acetamide (14 g) in dimethyl sulfoxide (126 mL) was heated to 50°C to dissolve the compound. The resulting solution was filtered at hot, followed by washing with dimethyl sulfoxide (14 mL). The filtrate was heated to 65°C and water (70 mL) was added thereto. The mixture was stirred at 70°C for 2 hours, then cooled to 20°C over 5 hours, and allowed to stand at 20°C for 16 hours. The precipitated crystals were filtered, washed with dimethyl sulfoxide/water = 2/1 (14 mL) and water (14 mL x 2), and dried under reduced pressure at 60°C for 16 hours to obtain a compound of the present invention (13.3 g) having the following physical properties.

It was confirmed by gas chromatography that dimethyl sulfoxide as a residual solvent remained in only 0.2% content in the crystals of the compound of the present invention obtained by the above method. This level of the residual amount was not significant at all in view of medicinal regulation.

### Example 1 (5):

A suspension of 2-[5-amino-6-oxo-2-phenyl-1,6-dihydro-1-pyrimidyl]-N-[1-(2-[5-t-butyl-1,3,4-oxadiazolyl]carbonyl)-2-(R,S)-methylpropyl]acetamide (14 g) in acetone/water = 9/1 (142.5 mL) was heated to 50°C to dissolve the compound. The resulting solution was filtered under heating. The filtrate was cooled to 20°C over 3 hours and stirred at 20°C for 1 hour. Water (112.5 mL) was added thereto, followed by stirring for 30 minutes. The mixture was stirred at 5°C overnight. The precipitated crystals were filtered, washed with acetone/water = 1/1 (5 mL × 2) and water (5 mL), and dried under reduced pressure at 60°C overnight to obtain a Compound of the present invention (13.96 g) having the following physical properties.

It was confirmed by gas chromatography that acetone as a residual solvent remained in only 0.2% content in the crystals of the compound of the present invention obtained by the above method. This level of the residual amount was not significant at all in view of medicinal regulation.

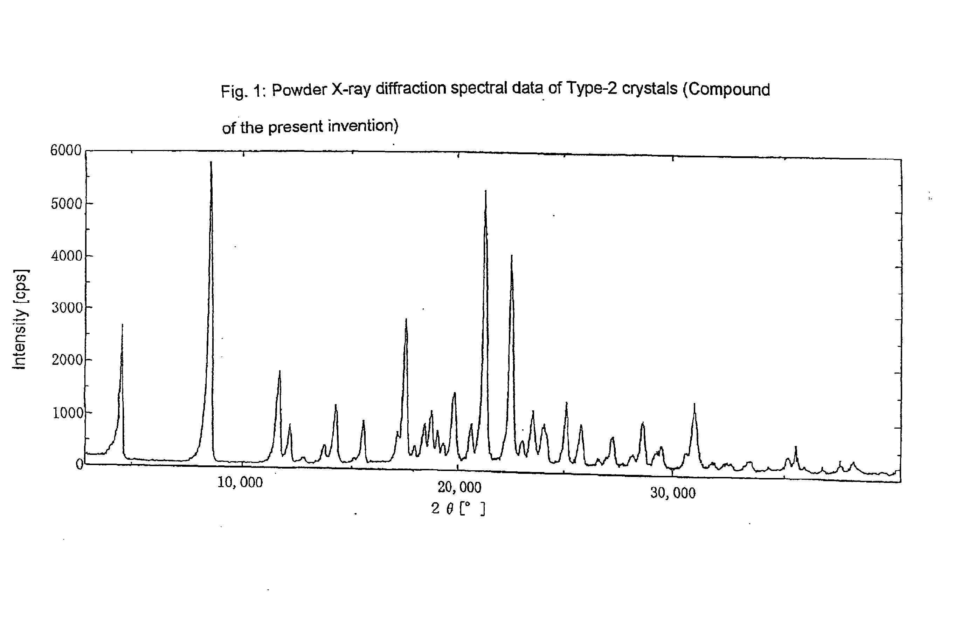

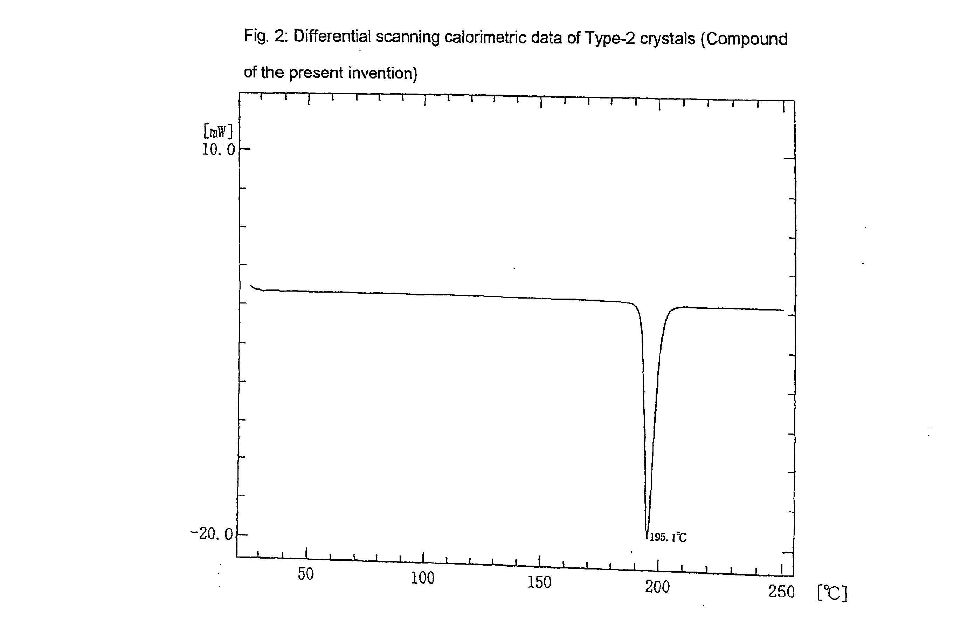

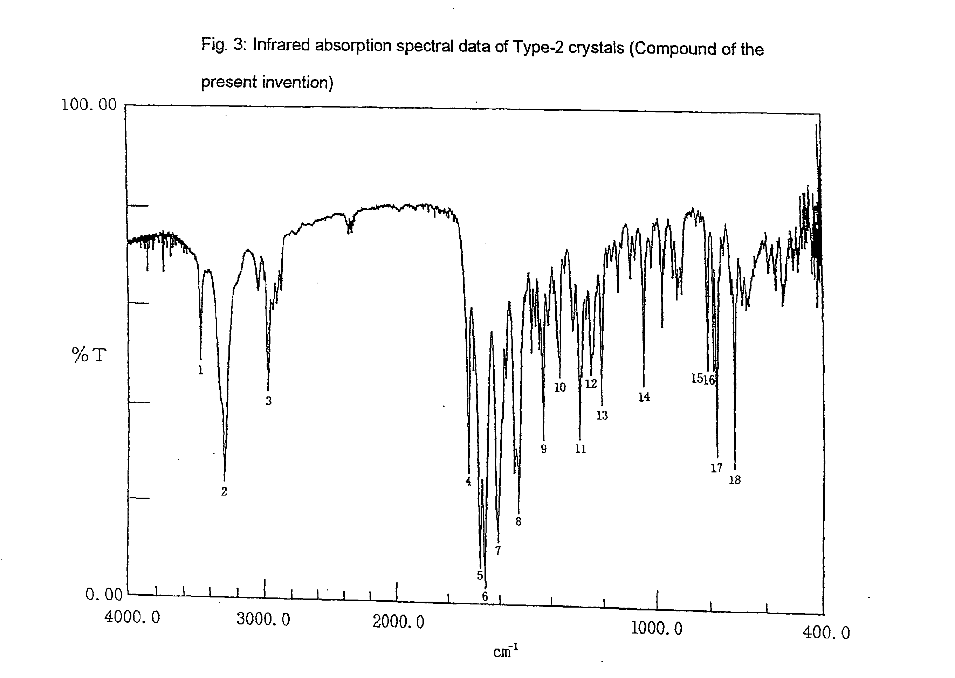

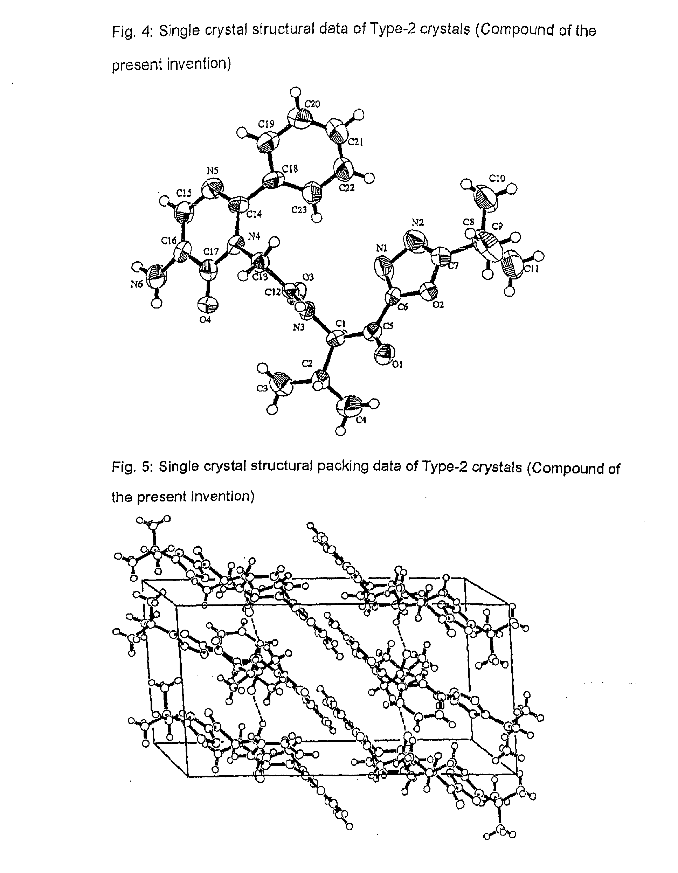

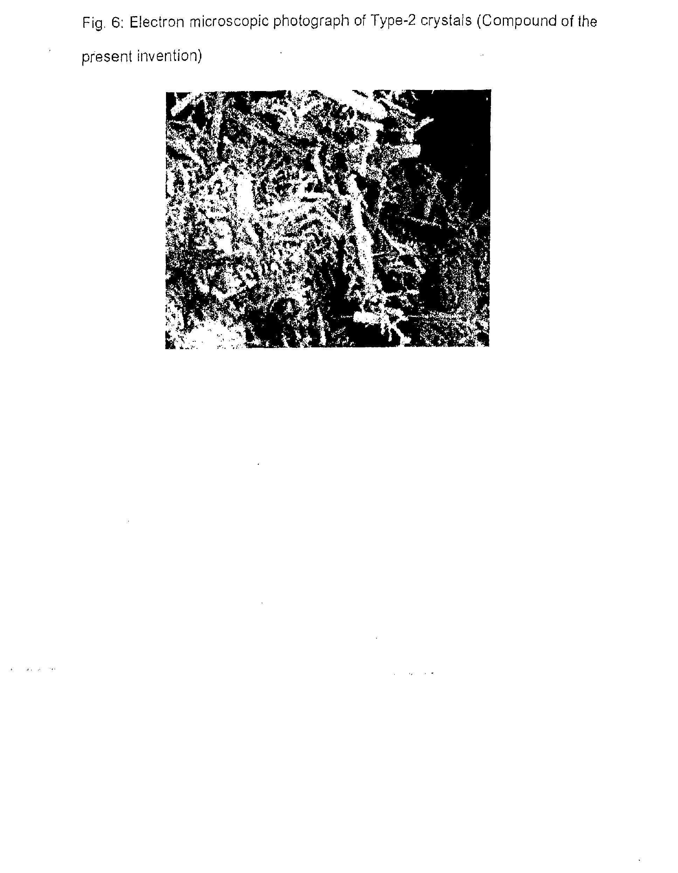

Powder X-ray diffraction spectral data of the crystals of the compound of the present invention thus obtained were shown in Fig. 1, differential scanning calorimetric data thereof were shown in Fig. 2, infrared absorption spectral data thereof were shown in Fig. 3, structural analysis data on single crystal X-ray diffraction spectrum thereof were shown on Fig. 4 and Fig. 5, and electron microscopic photograph thereof were shown in Fig. 6.

### (1) Powder X-ray diffraction spectral data

### [Measuring conditions]

Apparatus: Powder X-ray diffraction apparatus RAD-2C, manufactured by K.K. Rigaku,
Target: Cu
Filter: not employed
Voltage: 40 kV
Current: 20 mA
Scanning speed: 2.0°/minute

### [Results]

The results were shown in Table 2.

**Table 2**

| Diffraction angle (2θ) | Half band width | Relative intensity |
|---|---|---|
| 4.60 | 0.09 | 46 |
| 8.52 | 0.14 | 100 |
| 11.64 | 0.17 | 32 |
| 12.16 | 0.19 | 14 |
| 14.28 | 0.19 | 19 |
| 15.52 | 0.14 | 16 |
| 17.48 | 0.19 | 47 |
| 18.72 | 0.12 | 18 |
| 19.82 | 0.21 | 25 |
| 20.62 | 0.14 | 15 |
| 21.30 | 0.24 | 91 |
| 22.54 | 0.17 | 66 |
| 23.08 | 0.12 | 10 |
| 23.56 | 0.07 | 20 |
| 24.08 | 0.07 | 16 |
| 24.20 | 0.12 | 13 |
| 25.10 | 0.17 | 22 |
| 27.30 | 0.12 | 12 |
| 28.62 | 0.14 | 17 |
| 31.00 | 0.07 | 24 |

### (2) Differential scanning calorimetric data

### [Measuring conditions]

Apparatus: DSC-50 manufactured by Shimadzu Corporation,
Sample amount: 4.54 mg
Sample cell aluminum cell
Flow rate of nitrogen gas: 20 mL/minute
Temperature elevation rate: 10°C/minute

### [Results]

As a result, it was found that the product has an endothermic peak at around 195°C.

### (3) Infrared absorption spectral data

### [Measuring conditions]

Apparatus: JASCO VALOR-lll manufactured by JASCO Corporation
Resolution: 1 cm⁻¹,KBr method.
Scanning number of times: 16 times

### [Results]

IR (KBr): ν 3474, 3298, 2976, 1721, 1676, 1658, 1606, 1526, 1431, 1366, 1291, 1247, 1208, 1048, 809, 787, 777, 713cm⁻¹.

### (4) Structural analysis data on single crystal X-ray diffraction spectrum

### [Measuring conditions]

Apparatus: Single crystal X-ray diffraction apparatus AFC-5R, manufactured by K. K. Rigaku,
Target: Cu
Filter: Ni filter
Voltage: 40 kV
Current: 150 mA
Scanning speed: 8.0°/min

### [Results]

Crystallographic data were as follows:
Lattice constants: a=9.694Å, b=12.226 Å, c=19.351Å, β=95.42°,
Space group: P21/a
R factor: R=0.122

### (5) Electron microscopic photograph

### [Measuring conditions]

Apparatus: S-2460N manufactured by Hitachi Ltd.
Magnification: 250 magnifications

### [Results]

The crystals are regarded as needle crystals.

### Comparative Example 1: Process for producing 2-[5-amino-6-oxo-2-phenyl-1,6-dihydro-1-pyrimidyl]-N-[1-(2-[5-t-butyl-1,3,4-oxadiazolyl]carbonyl)-2-(R,S)-methylpropyl]acetamide (method described in Example 113 of WO98/24806)

Anisole (0.65 mL) was added to a solution of 2-[5-benzyloxycarbonylamino-6-oxo-2-phenyl-1,6-dihydro-1-pyrimidyl]-N-[1-(2-[5-t-butyl-1,3,4-oxadiazolyl]carbonyl)-2-(R,S)-methylpropyl]acetamide (586 mg) in dichloromethane (16 mL). A solution of aluminum chloride (800 mg) in nitromethane (8 mL) was added to the mixture at 0°C. The reaction mixture was stirred at 0°C for 1.5 hours. Ice-water was added to the reaction mixture, followed by extraction with ethyl acetate. The extract was washed with saturated aqueous sodium chloride solution, dried over anhydrous magnesium sulfate, and concentrated. The resulting residue was purified by silica gel column chromatography (hexane/ethyl acetate = 1/2 → ethyl acetate) and the resulting solid was dried *in vacuo* at room temperature for overnight to obtain the title compound having the following physical properties (hereinafter abbreviated as "Compound of Comparative Example 1") (356 mg) as a solid.

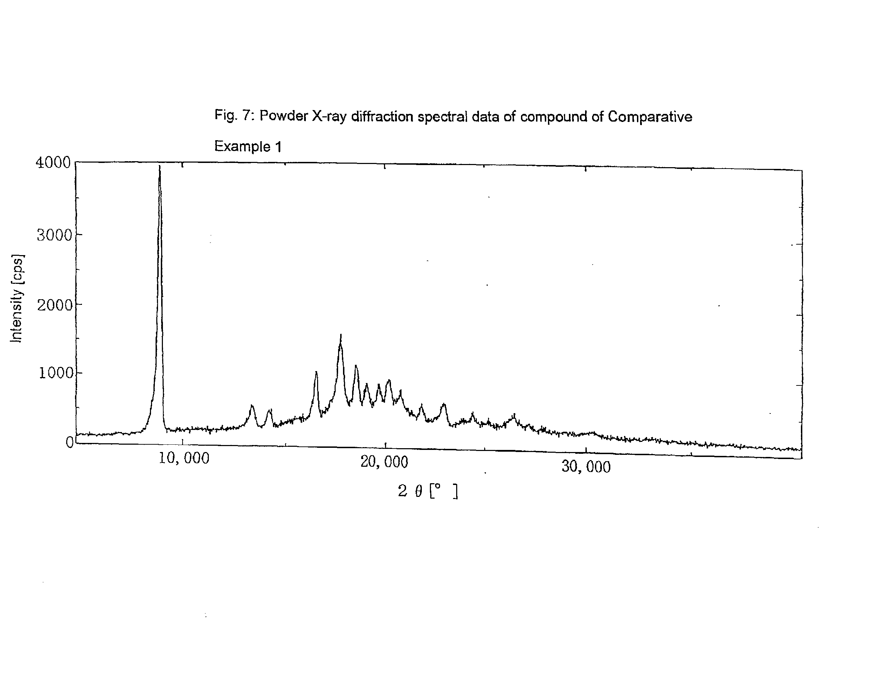

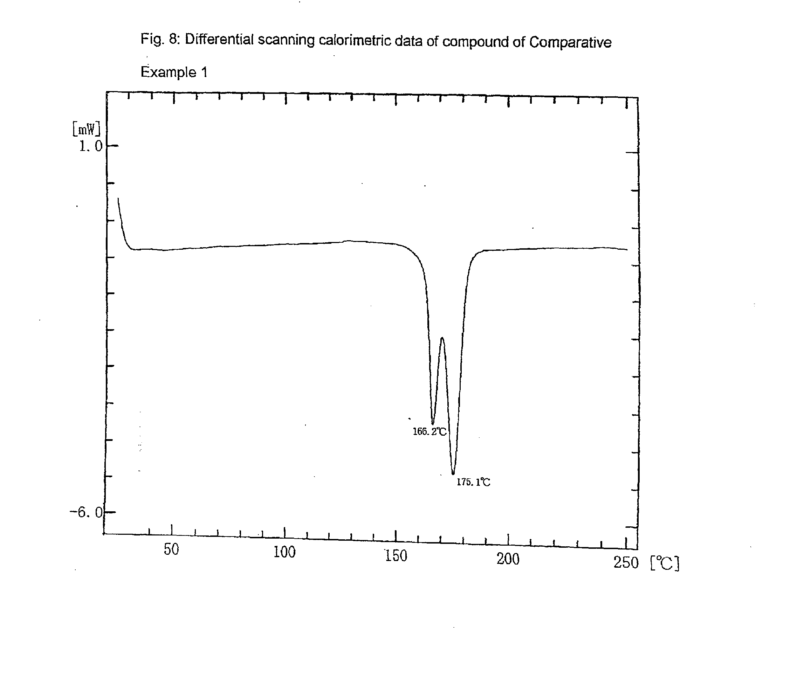

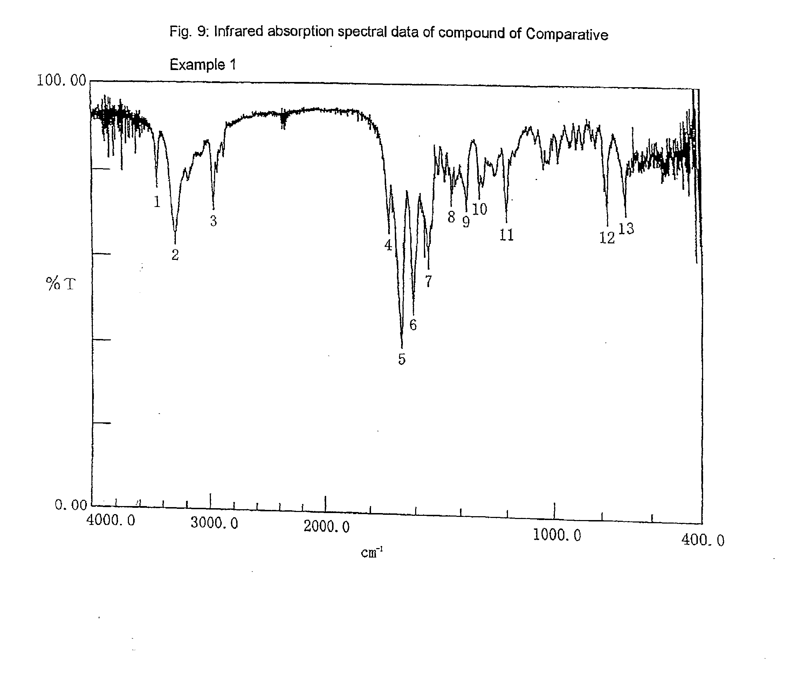

Powder X-ray diffraction spectral data, differential scanning calorimetric data, and infrared absorption spectral data of the compound obtained by the above method were shown in Fig. 7, Fig. 8, and Fig. 9, respectively.

### (1) Powder X-ray diffraction spectral data

### [Measuring conditions]

Apparatus: Powder X-ray diffraction apparatus RAD-2C, manufactured by K.K. Rigaku,
Target: Cu
Filter: not employed.
Voltage: 40 kV
Current: 20 mA
Scanning speed: 2.0°/minute

### [Results]

The results were shown in Table 3.

**Tables 3**

| Diffraction angle (2θ) | Half band width | Relative intensity |
|---|---|---|
| 8.94 | 0.17 | 100 |
| 13.26 | 0.07 | 15 |
| 16.30 | 0.07 | 16 |
| 16.52 | 0.12 | 27 |
| 17.76 | 0.07 | 41 |
| 18.06 | 0.07 | 17 |
| 18.50 | 0.21 | 30 |
| 18.90 | 0.07 | 21 |
| 19.04 | 0.09 | 23 |
| 19.16 | 0.07 | 20 |
| 19.26 | 0.07 | 16 |
| 19.64 | 0.09 | 23 |
| 20.08 | 0.12 | 23 |
| 20.18 | 0.09 | 25 |
| 20.46 | 0.07 | 17 |
| 20.78 | 0.07 | 21 |
| 21.16 | 0.09 | 15 |
| 23.02 | 0.07 | 15 |

### (2) Differential scanning calorimetric data

### [Measuring conditions]

Apparatus: DSC-50 manufactured by Shimadzu Corporation
Sample amount: 4.84 mg
Sample cell aluminum cell
Flow rate of nitrogen gas: 20 mL/minute
Temperature elevation rate: 10°C/minute

### [Results]

As a result, it was found that the product has endothermic peaks at around 166°C and 175°C.

### (3) Infrared absorption spectral data

### [Measuring conditions]

Apparatus: JASCO VALOR-lll manufactured by JASCO Corporation
Resolution: 1 cm⁻¹, KBr method
Scanning number of times: 16 times

### [Results]

IR (KBr): ν 3452, 3300, 2972, 1716, 1662, 1610, 1543, 1437, 1372, 1318, 1202, 777, 704 cm⁻¹ .

### Comparative Example 2: Process for producing Type-1 crystal of 2-[5-amino-6-oxo-2-phenyl-1, 6-dihydro-1-pyrimldyl]-N-[1-(2-[5-t-butyl-1,3,4-oxadiazolyl]carbonyl)-2-(R,S)-methylpropyl]acetamide

2-[5-Amino-6-oxo-2-phenyl-1,6-dihydro-1-pyrimidyl]-N-[1-(2-[5-t-butyl-1,3,4-oxadiazolyl]carbonyl)-2-(R, S)-methylpropyl]acetamide (1 g) was suspended in a mixed solution of ethanol (4 mL) and tetrahydrofuran (4 mL). The mixture was refluxed to dissolve the compound. After dissolution, the solution was filtered at hot. The filtrate was stirred overnight while allowing to cool. The precipitated crystals were filtered and dried *in vacuo* at 60°C overnight to obtain Compound of the title compound having the following physical properties (hereinafter abbreviated as "Compound of Comparative Example 2") (445 mg).

It was confirmed by NMR that tetrahydrofuran remained in 6.1 % content in Type-1 crystal obtained by the above method.

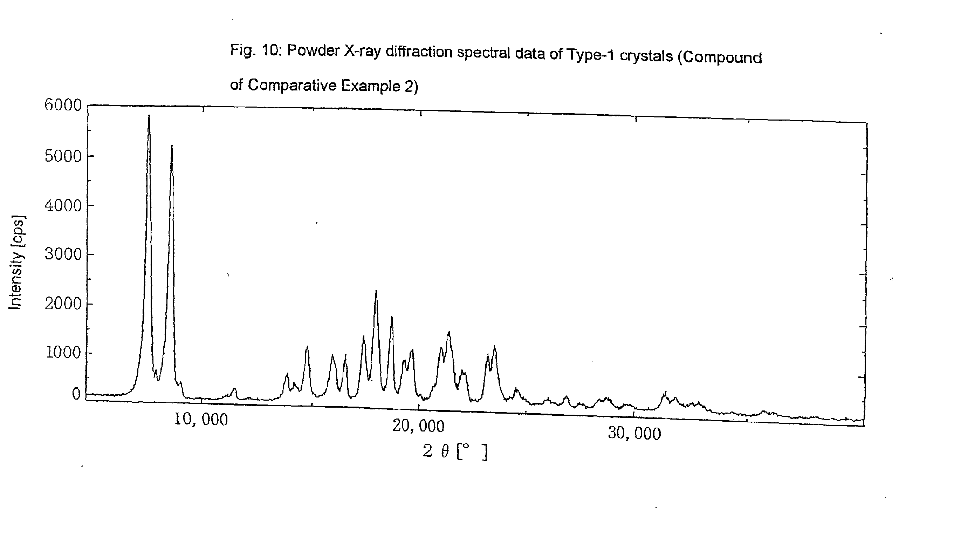

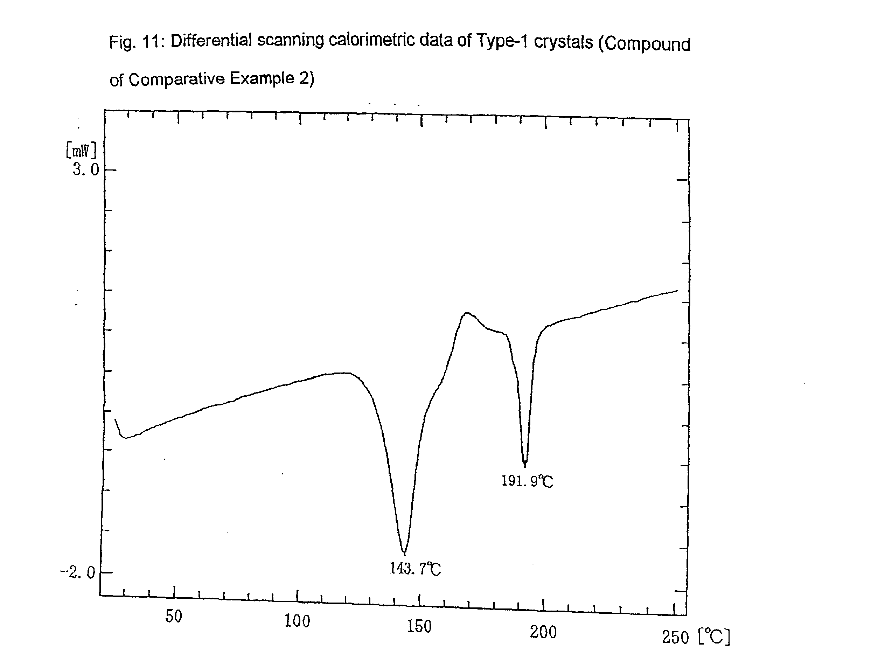

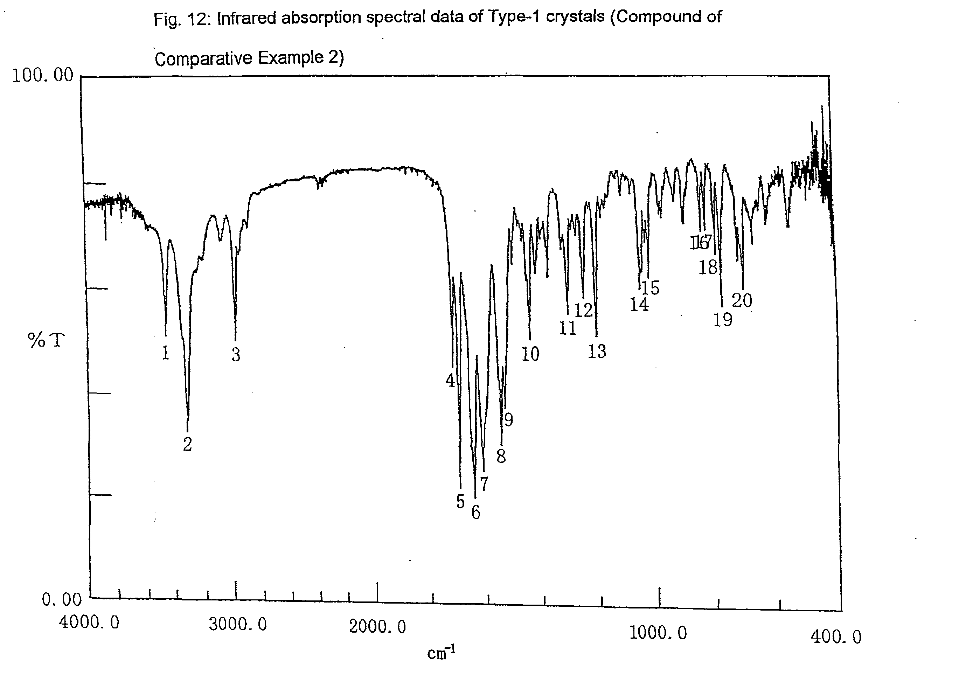

Powder X-ray diffraction spectral data, differential scanning calorimetric data, and infrared absorption spectral data of Type-1 crystal obtained by the above method were shown in Fig. 10, Fig. 11, and Fig. 12, respectively.

### (1) Powder X-ray diffraction spectral data

### [Measuring conditions]

Apparatus: Powder X-ray diffraction apparatus RAD-2C, manufactured by K.K. Rigaku,
Target: Cu
Filter: not employed
Voltage: 40 kV
Current: 20 mA
Scanning speed: 2.0°/minute

### [Results]

The results were shown in Table 4.

**Table 4**

| Diffraction angle (2θ) | Half band width | Relative intensity |
|---|---|---|
| 7.64 | 0.19 | 100 |
| 8.64 | 0.21 | 90 |
| 14.74 | 0.21 | 21 |
| 15.92 | 0.12 | 19 |
| 16.54 | 0.14 | 19 |
| 17.36 | 0.12 | 26 |
| 17.92 | 0.14 | 41 |
| 18.70 | 0.14 | 32 |
| 19.26 | 0.07 | 16 |
| 19.32 | 0.07 | 17 |
| 19.66 | 0.17 | 21 |
| 20.98 | 0.14 | 21 |
| 21.34 | 0.14 | 27 |
| 21.42 | 0.07 | 26 |
| 21.58 | 0.07 | 18 |
| 23.14 | 0.07 | 17 |
| 23.22 | 0.14 | 20 |
| 23.56 | 0.14 | 23 |

### (2) Differential scanning calorimetric data

### [Measuring conditions]

Apparatus: DSC-50 manufactured by Shimadzu Corporation
Sample amount: 2.34 mg
Sample cell: aluminum cell
Flow rate of nitrogen gas: 20 mL/minute
Temperature elevation rate: 10°C/minute

### [Results]

As a result, it was found that the product has endothermic peaks at around 143°C and 191°C.

### (3) Infrared absorption spectral data

### [Measuring conditions]

Apparatus: JASCO VALOR-lll manufactured by JASCO Corporation
Resolution: 1 cm⁻¹, KBr method.
Scanning number of times: 16 times

### [Results]

IR (KBr): ν 3459, 3317, 2973, 1716, 1694, 1641, 1609, 1544, 1529, 1437, 1302, 1247, 1204, 1051, 1016, 839, 825, 790, 772, 701 cm⁻¹.

### Difference between the compound of the present invention and the compound of Comparative Example 1 or the compound of Comparative Example 2

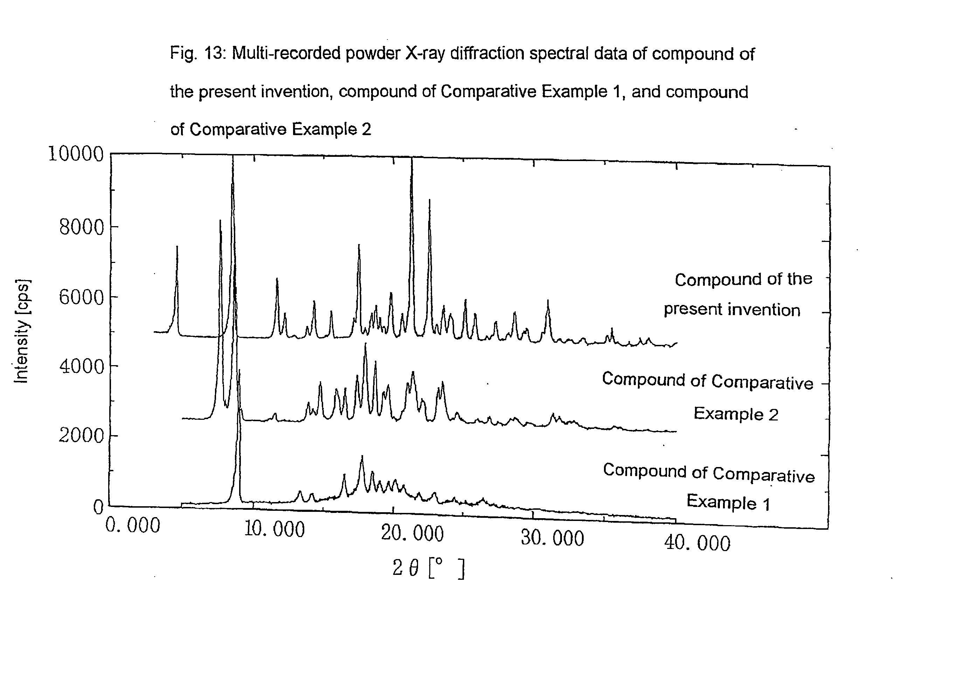

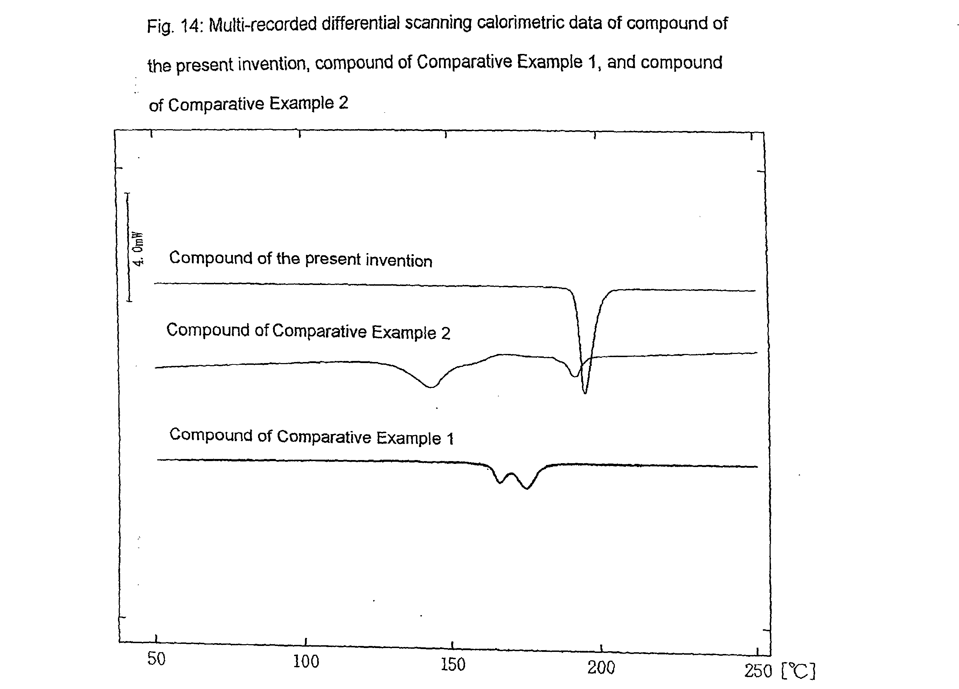

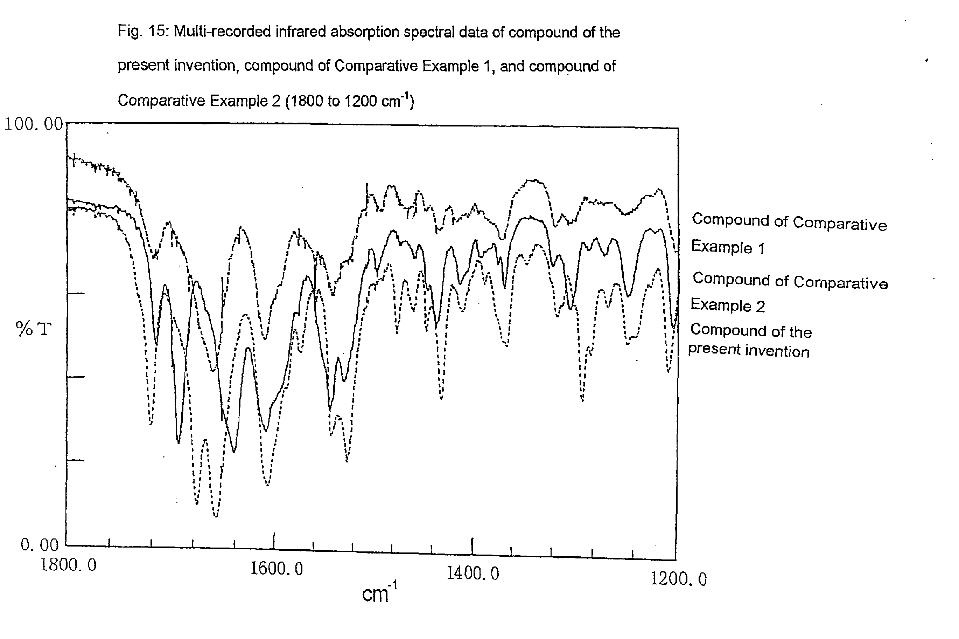

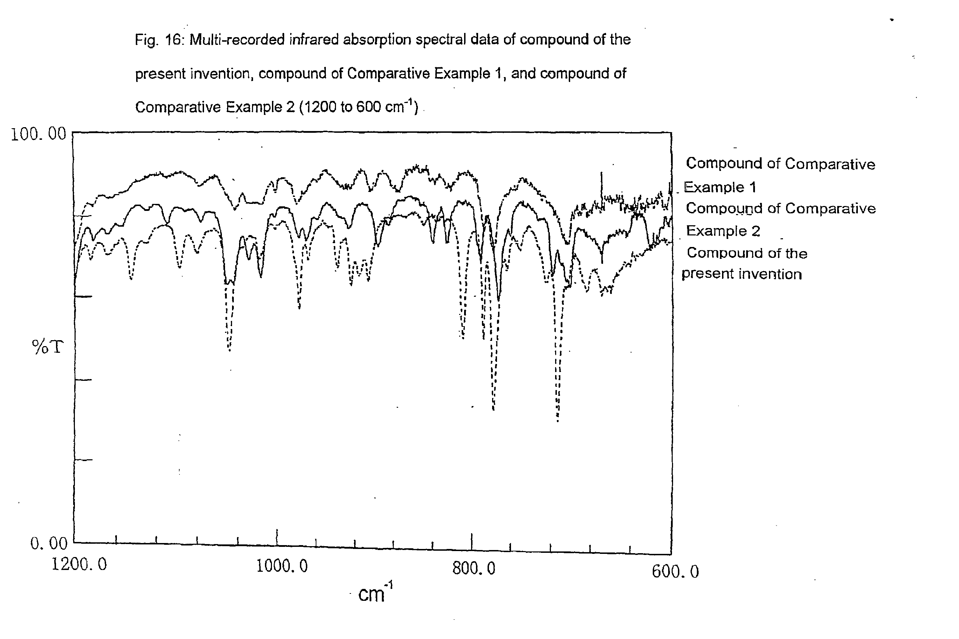

Multi-recorded powder X-ray diffraction spectral data of the compound of the present invention, the compound of Comparative Example 1, and the compound of Comparative Example 2 were shown in Fig. 13, multi-recorded differential scanning calorimetric data thereof were shown in Fig. 14, and multi-recorded infrared absorption spectral data thereof were shown in Fig. 15 and Fig. 16.

As a result, it was found that the compound of the present invention is entirely different from the compound of Comparative Example 1 and the compound of Comparative Example 2 in powder X-ray diffraction spectral data, differential scanning calorimetric data, and infrared absorption spectral data.

### Solubility of Compound of the present invention in various solvents

### [Measuring operations]

The compound of the present invention was added to various solvents so that a saturated state was achieved. Each mixture was stirred for 30 seconds by means of a laboratory mixer and then allowed to stand for 4 minutes and 30 seconds. The stirring and standing operations were repeated seven times. The mixture was centrifuged at 3000 rpm for 15 minutes and isoamyl 4-hydroxybenzoate was added as an internal standard to the resulting supernatant. Furthermore, acetonitrile was added to the supernatant to form a sample solution. A portion (8 µL) of each solution was analyzed using high performance liquid chromatography (HPLC) under the following conditions to determine its concentration by the internal standard method, and thereby solubility was calculated. Additionally, pH at the dissolution was measured.

### [Measuring conditions]

### HPLC measuring conditions

Column: CAPCELLPACK C8 UG120 (4.6 mm i.d. x 150 mm, No. BOAB1013)
Temperature: 25°C
Eluent: 20 mmol/L aqueous potassium dihydrogen phosphate solution (pH 8.0,
sodium hydroxide)/acetonitrile = 65/35
Flow rate: 1.0 mL/minute
UV: 235 nm

### [Results]

The results obtained were shown in Table 5.

**Table 5**

| Solvent | Solubility | pH |
|---|---|---|
| Methanol | 33.6 mg/mL | - |
| Acetonitrile | 19.1 mg/mL | - |
| Ethanol | 7.29 mg/mL | - |
| Octanol | 0.71 mg/mL | - |
| Purified water | 0.073 mg/mL | 7.3 |
| Buffer pH 3.0 | 0.122 mg/mL | 3.0 |
| Buffer pH 5.0 | 0.104 mg/mL | 5.0 |
| Buffer pH 7.0 | 0.094 mg/mL | 7.0 |
| Buffer pH 9.0 | 0.045 mg/mL | 8.6 |

As the buffer (buffer solution) in Table 5, Briton-Robinson Buffer having an ionic strength of 0.2 was used.

### Stability test of the compound of the present invention

### [Measuring operations]

The compound of the present invention (about 50 mg) was weighed in a test tube and stored under the following conditions. The stored sample was analyzed using high performance liquid chromatography (HPLC) to calculate its residual ratio by the internal standard method.
Condition 1: 60°C, sealed, 1 month;
Condition 2: 40°C, open, 75%RH (relative humidity), 3 months;
Condition 3: 40°C, sealed, 6 months;
Condition 4: 25°C, open, 60%RH (relative humidity), 12 months;
Condition 5: 5°C, sealed, 12 months;
The HPLC measurement was carried out under the same conditions as in Example 2 described above.

### [Results]

The results obtained were shown in Table 6.

**Table 6**

| Conditions | Duration | Residual ratio |
|---|---|---|
| 60°C, sealed | 1 month | 100.1% |
| 40°C, open, 75%RH | 3 months | 100.0% |
| 40°C, sealed | 6 months | 99.9% |
| 25°C, open, 60%RH | 12 months | 99.5% |
| 5°C, sealed | 12 months | 99.5% |

From Table 6, it was confirmed that the compound of the present invention is sufficiently stable.

### Industrial Applicability

The compound of the present invention has an inhibitory activity against an elastase. The inhibitory activity against an elastase was confirmed by the screening system described in WO98/24806.

### [Toxicity]

It was confirmed that the compound of the present invention has a sufficiently low toxicity and can be employed as a medicament with sufficient safety.

### [Application to medicaments]

The compound of the present invention is a compound having an inhibitory activity against an elastase and is useful for treating and/or preventing diseases caused by abnormal increase of elastin decomposition, decomposition of collagen fibers, and/or proteoglycan decomposition by elastase in mammalian, especially human, for example, arthritis, periodontal disease, nephritis, dermatitis, psoriasis, cystic fibrosis, chronic bronchitis, atherosclerosis, Alzheimer's disease, organ transplantation, corneal ulcers, malignant tumors, and the like.

In order to use the Compound of the present invention for the purpose described above, the compound is usually administered systemically or locally, orally or parenterally.

The doses to be administered may vary depending upon age, body weight, symptom, therapeutic effect, the route of administration, and the duration of the treatment. In the human adult, the doses per person at a time are generally from 1 mg to 1000 mg, by oral administration, once to several times per day, or from 1 mg to 100 mg, by parenteral administration, once to several times per day.

As described above, the doses to be administered depend upon various conditions. Therefore, there are cases wherein doses lower than the ranges specified above may be enough or doses or greater than the range may be required.

The compound of the present invention is administered in the form of solid compositions for oral administration or parenteral administration.

Solid compositions for oral administration include tablets, pills, capsules, dispersible powders, granules, and the like.

Capsules include hard capsules and soft capsules.

In such solid compositions, one or more active substances may be admixed with at least one inert diluent, e.g. lactose, mannitol, glucose, hydroxypropyl cellulose, microcrystalline cellulose, starch, polyvinyl pyrrolidone, or magnesium metasilicate aluminate. The composition may contain, in addition to the inert diluent, lubricating agents such as magnesium stearate, disintegrating agents such as cellulose calcium glycolate, stabilizing agents such as lactose, agents to assist dissolution such as glutamic acid or aspartic acid, according to conventional methods. The tablets or pills may, if necessary, be coated with a film of a gastric or enteric soluble substance such as sugar, gelatin, hydroxypropyl cellulose or hydroxypropylmethyl cellulose phthalate, or be coated with two or more layers. Furthermore, capsules of an absorbable material such as gelatin may be also included.

Solid compositions for parenteral administration include suppositories for rectal administration and pessaries for vaginal administration, which comprise one or more of the active compounds and are prescribed by conventional methods.

### Formulation Example 1

The following components were admixed by a conventional method and punched out to give 100 tablets containing 50 mg of an active ingredient per tablet.

| | |
|---|---|
| Type-2 crystals of 2-[5-amino-6-oxo-2-phenyl-1,6-dihydro-1 -pynmidyl]-N-[1(2-[5-t-butyl-1,3,4-oxadiazolyl]carbonyl)-2-(R,S)-methylpropyl]acetamide (Compound of the present invention) | .....5.0g |
| Carboxymethyl cellulose calcium (disintegrating agent) | .....0.2 g |
| Magnesium stearate (lubricant) | .....0.1 g |
| Microcrystalline cellulose | .....4.7 g |

## Claims

1. A Type-2 crystal of 2-[5-amino-6-oxo-2-phenyl-1,6-dihydro-1-pyrimidyl]-N-[1-(2-[5-t-butyl-1,3,4-oxadiazolyl]carbonyl)-2-(R,S)-methylpropyl]acetamide having diffraction angles (2θ), half band widths, relative intensities shown in Table 1 in a powder X-ray diffraction spectrum obtained using Cu-Kα ray:
**Table 1**
| Diffraction angle (2θ) | Half band width | Relative intensity |
|---|---|---|
| 4.60 | 0.09 | Strong |
| 8.52 | 0.14 | Slightly strong |
| 11.64 | 0.17 | Medium |
| 12.16 | 0.19 | Medium |
| 14.28 | 0.19 | Medium |
| 15.52 | 0.14 | Medium |
| 17.48 | 0.19 | Strong |
| 18.72 | 0.12 | Medium |
| 19.82 | 0.21 | Medium |
| 20.62 | 0.14 | Medium |
| 21.30 | 0.24 | Slightly strong |
| 22.54 | 0.17 | Strong |
| 23.08 | 0.12 | Medium |
| 23.56 | 0.07 | Medium |
| 24.08 | 0.07 | Medium |
| 24.20 | 0.12 | Medium |
| 25.10 | 0.17 | Medium |
| 27.30 | 0.12 | Medium |
| 28.62 | 0.14 | Medium |
| 31.00 | 0.07 | Medium |

2. A process for producing the compound according to claim 1, which comprises recrystallizing a crude purified product of 2-[5-amino-6-oxo-2-phenyl-1 ,6-dihydro-1-pyrimidyl]-N-[1-(2-[5-t-butyl-1 3,4-oxadiazolyl]carbonyl)-2-(R,S)-methylpropyl]acetamide from a mixed solvent comprising water and one or more solvents selected from the group consisting of methanol, ethanol, dimethylformamide, dimethyl sulfoxide, acetone, and ethyl acetate.

3. A pharmaceutical composition comprising the compound according to claim 1 as an active ingredient.

4. An elastase inhibitor comprising the compound according to claim 1 as an active ingredient.

5. A solid composition comprising the compound according to claim 1 as an active ingredient.
